Europäisches Patentamt

European Patent Office

Office européen des brevets

(19)

(11) EP 0 725 075 A1

(12) DEMANDE DE BREVET EUROPEEN

(43) Date de publication:
07.08.1996 Bulletin 1996/32

(51) Int Cl.$^6$: C07K 5/03, A61K 38/07

(21) Numéro de dépôt: 96400229.9

(22) Date de dépôt: 02.02.1996

(84) Etats contractants désignés:
CH DE GB IT LI

(30) Priorité: 06.02.1995 FR 9501328

(71) Demandeur: COMMISSARIAT A L'ENERGIE ATOMIQUE
75015 Paris (FR)

(72) Inventeurs:
• Dive, Vincent
F-94300 Vincennes (FR)
• Jiracek, Jiri
Parha 8 (CZ)
• Yiotakis, Athanasios
GR-15342 Ag Paraskevi/Athenes (GR)

(74) Mandataire: Des Termes, Monique et al
Société Brevatome
25, rue de Ponthieu
75008 Paris (FR)

(54) Dérivés de peptides comportant une acide phosphinique utilisables comme inhibiteur de l'endopeptidase à zinc 24-15

(57) L'invention concerne de nouveaux dérivés de peptides utilisables comme inhibiteur de l'endopeptidase à zinc 24-15.

Ces dérivés de peptides comportent la séquence d'acides aminés suivante :

- Phe $\Psi(PO_2CH_2)$-$_{(L,D)}$Xaa'-Yaa'-Zaa'-

dans laquelle

$\Psi(PO_2CH_2)$ indique que la liaison peptidique (CONH) a été remplacée par la liaison phosphinique $(PO_2CH_2)$,

Xaa' et Zaa' qui peuvent être identiques ou différents, représentent chacun un acide aminé naturel ou un pseudo-acide aminé, et

Yaa' représente Arg ou Lys.

A titre d'exemple de tels dérivés, on peut citer ceux de formule :

Z-$_{(L,D)}$Phe $\Psi(PO_2CH_2)$-$_{(L,D)}$-Gly-Arg-MetOH

Z-$_{(L,D)}$Phe $\Psi(PO_2CH_2)$-$_{(L,D)}$-Ala-Arg-MetOH

Z-$_{(L,D)}$Phe $\Psi(PO_2CH_2)$-$_{(L,D)}$-Ala-Arg-PheOH

avec Z représentant le groupe benzyloxycarbonyle.

EP 0 725 075 A1

**Description**

La présente invention a pour objet de nouveaux dérivés de peptides utilisables comme inhibiteur de l'endopeptidase à zinc EC.3.4.24-15.

De façon plus précise, elle concerne des dérivés de peptides qui sont des inhibiteurs puissants et sélectifs de l'endopeptidase 24-15, tout en étant inactifs vis-à-vis d'autres peptidases à zinc telles que l'endopeptidase 24-16, l'enzyme de conversion de l'angiotensine, l'endopeptidase 24-11, les amino peptidases M et L et les carboxypeptidases A et B.

L'obtention d'inhibiteurs de protéases à zinc est un enjeu pharmacologique d'importance. En effet, par le rôle qu'elles jouent chez les mammifères dans le métabolisme des protéines et des peptides, de nombreuses métalloprotéases à zinc sont impliquées dans des fonctions physiologiques importantes et peuvent être à l'origine de diverses pathologies. Au niveau du système nerveux central, mais aussi des systèmes plus périphériques, un certain nombre d'endopeptidases à zinc (endopeptidases 24-11, 24-15 et 24-16) interviennent dans la dégradation ou la maturation de nombreux neuropeptides. Au niveau du système cardio-vasculaire, les enzymes de conversion de l'angiotensine et de l'endothéline jouent un rôle essentiel dans la régulation de la pression artérielle. On trouve également des métalloprotéases à zinc dont l'activité est associée aux maladies du vieillissement et au développement des métastases cancéreuses (collagénase, élastase, gélatinase, stromélysine). D'autre part, de telles métalloprotéases ont pu être identifiées dans certains cas comme étant étroitement associées à la virulence de certains microorganismes (oxines botuluniques et tétaniques, hémagglutinine cholérique, pseudomonas aéruginosa, maladies parodontales dues aux bactéries collagénolytiques ).

Il serait donc intéressant de disposer d'inhibiteurs spécifiques de ces protéases dans des buts thérapeutiques.

Différents groupes dans le monde se sont intéressés à ce problème et ont développé une approche rationnelle pour synthétiser de tels inhibiteurs. Celle-ci repose sur une propriété fondamentale des métalloprotéases à zinc: la présence dans leur site actif d'un atome de zinc participant à la catalyse de l'hydrolyse de la liaison peptidique.

Globalement cette stratégie consiste à synthétiser des peptides analogues aux substrats de ces protéases mais dans lesquels on remplace la liaison peptidique (CO-NH) clivée par ces protéases par un groupement chimique présentant, d'une part, de bonnes analogies structurales et électroniques avec la liaison peptidique à l'état de transition, et capable, d'autre part, d'interagir fortement avec l'atome de zinc présent dans le site actif de ces protéases.

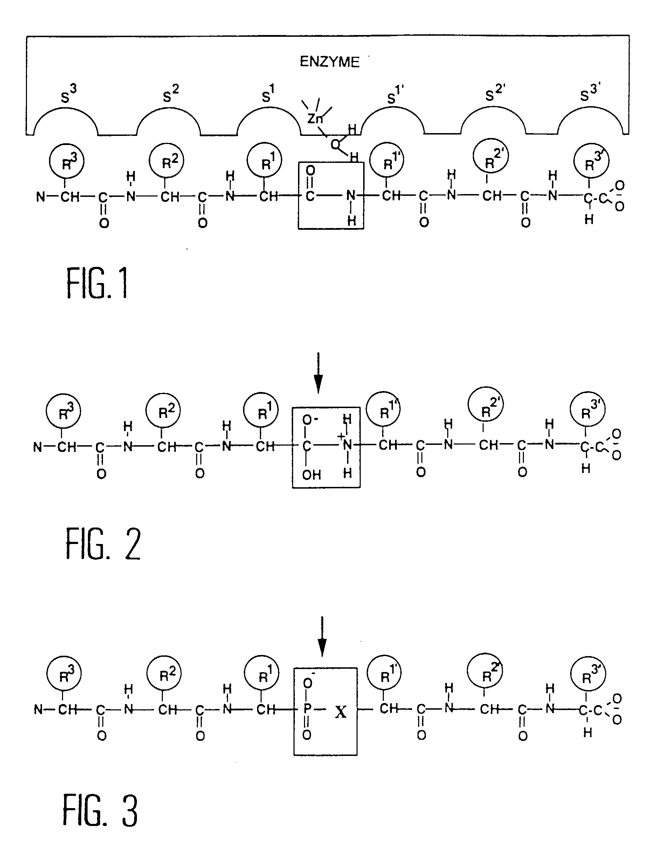

Sur les figures 1 à 3 annexées, on a illustré l'état fondamental d'un substrat peptidique de ces enzymes, puis son état de transition (figure 2) et l'état de transition (figure 3) d'un inhibiteur constitué par un analogue du substrat peptidique dans lequel la liaison CO-NH clivée par l'enzyme a été remplacée par une liaison $PO_2$-X avec X représentant NH, O ou $CH_2$.

Sur la figure 1, $R^1$, $R^2$, $R^3$, $R^{1'}$, $R^{2'}$ et $R^{3'}$ représentent les chaînes latérales des acides aminés situés de part et d'autre de la liaison peptidique ou phosphinique impliquée dans la réaction avec l'enzyme.

Ainsi, pour de tels inhibiteurs, on a retenu jusqu'à présent des groupements chimiques du type phosphonamide (X=NH), phosphonique (X=O) ou phosphinique (X=$CH_2$), qui présentent de bonnes analogies structurales et électroniques avec la liaison peptidique à l'état de transition.

La ressemblance de ces inhibiteurs avec les substrats à l'état de transition confère en général à ces molécules des affinités tout à fait exceptionnelles.

L'introduction d'une liaison phosphonamide dans des substrats a été décrite dans FR-A-2 654 430 et elle s'est révélée très efficace pour parvenir à des inhibiteurs puissants de certaines protéases à zinc. Cependant, la stabilité chimique de la liaison phosphonamide dépend fortement des séquences d'acides animés encadrant cette liaison et malheureusement pour certaines séquences, on observe une hydrolyse très rapide de la liaison phosphonamide.

Le recours à une liaison du type phosphonate a été décrite par Kaplan et al dans Biochemistry, 30, 1991, p. 8165-8170 et a permis d'obtenir dans le cas précis de la carboxypeptidase A l'inhibiteur synthétique le plus puissant jamais reporté pour une enzyme (constante d'inhibition $K_i = 10^{-5}M$).

Des inhibiteurs contenant une liaison phosphinique (X = $CH_2$) ont été décrits dans FR-A-2 676 059 et se sont révélés très efficaces dans le cas des collagénases bactériennes. Dans EP-A-0 565 450, on a décrit des inhibiteurs contenant une liaison phosphonamide (X=NH), très efficaces vis-à-vis de l'endopeptidase 24.15, mais aussi très bons inhibiteurs de l'endopeptidase 24.16.

Selon la présente invention, on a développé de nouveaux dérivés de peptides comportant une liaison phosphinique qui sont puissants et sélectifs comme inhibiteurs de l'endopeptidase 24-15 ; ces produits sont en particulier inactifs vis-à-vis d'autres protéases, en particulier l'endopeptidase 24-16, l'enzyme de conversion de l'angiotensine, l'endopeptidase 24-11, les aminopeptidases M et L et les carboxypeptidases A et B. De plus, ces dérivés sont beaucoup plus stables du point de vue chimique que les peptides phosphonamidiques.

Selon l'invention, ces nouveaux dérivés de peptides comportent la séquence d'acides aminés suivante :

- Phe $\Psi(PO_2CH_2)$-$_{(L,D)}$Xaa'-Yaa'-Zaa'-

dans laquelle

$\Psi(PO_2CH_2)$ indique que la liaison peptidique (CONH) a été remplacée par la liaison phosphinique ($PO_2CH_2$),
Xaa' et Zaa' qui peuvent être identiques ou différents, représentent chacun un acide aminé naturel ou un pseudo-acide aminé, et
Yaa' représente Arg ou Lys.

Dans cette séquence, le groupe $PO_2CH_2$ est sous forme $PO_2^-$ comme il apparait sur la figure 3 ; il est donc associé à un contre-ion tel que $K^+$, $Na^+$ ou tout autre métal acceptable du point de vue pharmacologique. La nature du contre-ion n'a aucune importance car dans l'eau les groupements chargés sont dissociés.

Selon un mode particulier de réalisation de l'invention, ces dérivés de peptides répondent à l'une des formules :

$$Z\text{-}_{(L,D)}Phe\ \Psi(PO_2CH_2)\text{-}_{(L,D)}Xaa'\text{-}Arg\text{-}Zaa'OR^1 \tag{I}$$

et

$$Z\text{-}_{(L,D)}Phe\ \Psi(PO_2CH_2)\text{-}_{(L,D)}Xaa'\text{-}Lys\text{-}Zaa'OR^1 \tag{II}$$

dans lesquelles

Z représente un groupe protecteur conventionnel de la synthèse peptidique tel que le groupe acétyle ou benzyloxycarbonyle,
$\Psi(PO_2CH_2)$ indique que la liaison peptidique (CO-NH) a été remplacée par la liaison phosphinique ($PO_2CH_2$),
Xaa' et Zaa' qui peuvent être identiques ou différents, représentent chacun un acide aminé naturel ou un pseudo acide aminé, et
$R^1$ représente un atome d'hydrogène, $NH_4^+$ ou un métal pharmaceutiquement acceptable.

Dans les formules (I) et (II) données ci-dessus, les acides aminés utilisés pour Xaa' et Zaa' peuvent être des acides aminés naturels ou des pseudo-acides aminés.

Les acides aminés naturels peuvent être choisis parmi l'alanine, l'arginine, l'asparagine, l'acide aspartique, la cystéine, la glutamine, l'acide glutamique, la glycine, l'histidine, l'isoleucine, la leucine, la norleucine, la lysine, la méthionine, la phénylalanine, la proline, l'hydroxyproline, la sérine, la thréonine, le tryptophane, la tyrosine, la valine, la nitrophényl alanine, l'homo arginine, la thiazolidine et la déshydroproline.

Un pseudo acide aminé peut être défini comme un acide aminé dans lequel la fonction soit amino ou carbonyle a été remplacée par un autre groupement chimique.

Le métal utilisé pour $R^1$ peut être un métal alcalin tel que le sodium, le potassium ou le lithium.

Par ailleurs, comme il apparaît dans la formule, les acides aminés Phe et Xaa' peuvent être sous forme L ou D. Aussi, le dérivé de peptide de formule (I) ou (II) peut être constitué par un mélange de 4 diastéréoisomères ou par un seul de ces isomères.

De préférence, dans les formules (I) et (II) données ci-dessus, Xaa' représente Gly, Ala ou Leu car la présence d'un tel acide aminé au voisinage de la liaison phosphinique permet d'obtenir de très bons inhibiteurs.

Ces dérivés de peptides sont différents des dérivés de peptides décrits dans EP-A-0 565 450 pour lesquels on avait toujours au voisinage de la liaison phosphinique l'enchaînement Gly-Pro (ou analogue de Pro) ou Ala-Pro (ou analogue de Pro). Dans l'invention, on a remplacé le groupe proline (hydroxyproline, thiazolidine ou désydroproline) par un groupe arginine ou lysine qui permet d'atteindre une sélectivité très élevée pour l'endopeptidase 24-15 par rapport à l'endopeptidas 24.16.

Ainsi, dans ces dérivés, le choix de l'arginine (formule I) ou de la lysine (formule II) permet d'atteindre la spécificité voulue.

En effet, tout peptide contenant une liaison phosphinique peut être potentiellemnt un inhibiteur des différentes protéases appartenant à la famille des métalloprotéases à zinc. Cependant, en dehors des interactions de la liaison phosphinique avec l'atome de zinc du site actif, l'affinité du peptide dépend aussi des interactions entre les acides aminés de part et d'autre du motif phosphinique ($R^1$, $R^2$, $R^3$, $R^{1'}$, $R^{2'}$ et $R^{3'}$ sur la fig. 3) et les différents sous-sites ($S^1$, $S^2$, $S^3$, $S^{1'}$, $S^{2'}$ et $S^{3'}$ de la figure 1) du site actif de la protéase.

Selon l'invention, on a trouvé que dans le cas de l'endopeptidase 24-15, la présence d'arginine ou de lysine en position $R^{2'}$, conférait au peptide une affinité élevée pour les sous-sites de l'endopeptidase 24-15, et peu d'affinité pour les sous-sites d'autres protéases.

Selon l'invention, l'acide aminé représentant Zaa' a également beaucoup d'importance, car son choix permet d'optimiser l'interaction de l'inhibiteur avec l'endopeptidase 24-15, et de défavoriser son interaction vis-à-vis de l'endopeptidase 24-16 et d'autres protéases.

De préférence, selon l'invention Zaa' représente Met, Nle, Ala ou Phe.

Dans le cas des dérivés de formule II, on obtient aussi de bons résultats lorsque Zaa' représente Leu ou Ile.

Dans le cas du dérivé de formule I, on obtient les meilleurs résultats en ce qui concerne la puissance et la sélectivité de l'inhibiteur, lorsque Xaa' représente Ala, et Zaa' représente Met.

Dans le cas des dérivés de formule II, on obtient de très bons résultats lorsque Xaa' représente Ala et Zaa' représente Met ou Phe.

Ainsi, conformément à l'invention, on dispose d'inhibiteurs puissants et sélectifs de l'endopeptidase 24-15 de mammifère, ou thimet peptidase, qui sont par ailleurs de très mauvais inhibiteurs de l'endopeptidase 24-16. Ceci constitue un résultat très important, car les peptidases 24-15 et 24-16 ont des spécificités très proches et jusqu'à présent seuls étaient disponibles des inhibiteurs mixtes de ces deux peptidases comme il est décrit par H.Barelli et al dans Biochem. J., 1992, 287, pages 621-625 et dans EP-A-0 565 450.

Les dérivés de peptides de l'invention peuvent être préparés par des procédés classiques tels que celui décrit dans FR-A-2 676 059.

Toutefois, on préfère préparer les dérivés de formule (I) ou (II) par des procédés de synthèse en phase solide à partir des synthons de formule :

$$Z\text{-}_{(L,D)}Phe\Psi(PO(OR^3)CH_2)\text{-}_{(L,D)}Xaa'OH$$

dans laquelle Z, Phe, $\Psi$ et Xaa' ont les significations données ci-dessus, et $R^3$ représente le groupe adamantyle.

Aussi, l'invention a également pour objet un procédé de préparation d'un dérivé de peptide répondant aux formules (I) ou (II) données ci-dessus, qui consiste à coupler le dipeptide fixé sur une phase solide de formule : $NH_2$-Arg-Zaa'$R^2$ ou $NH_2$-Lys-Zaa'$R^2$ dans laquelle Zaa' a la signification donnée ci-dessus et $R^2$ est la phase solide, avec le synthon de formule :

$$Z\text{-}_{(L,D)}Phe\Psi(PO(OR^3)CH_2)\text{-}_{(L,D)}Xaa'OH$$

dans laquelle Z, $\Psi$, Xaa' et $R^3$ sont tels que définis ci-dessus, puis à séparer le dérivé de peptide de formule :

$$Z\text{-}_{(L,D)}Phe\Psi(PO(OR^3)CH_2)\text{-}_{(L,D)}Xaa'Arg\text{-}Zaa'R^2$$

ou

$$Z\text{-}_{(L,D)}Phe\Psi(PO(OR^3)CH_2)\text{-}_{(L,D)}Xaa'Lys\text{-}Zaa'R^2$$

de la phase solide $R^2$, et à éliminer le groupe $R^3$ par un traitement acide.

Dans le procédé décrit ci-dessus, les différentes étapes sont réalisées par des techniques classiques en utilisant les réactifs et solvants généralement employés dans la chimie des peptides.

L'invention a encore pour objet une composition pharmaceutique comprenant un inhibiteur de l'endopeptidase 24-15, caractérisée en ce que cet inhibiteur est un dérivé de peptide comportant la séquence d'acides aminés suivante :

$$\text{- Phe } \Psi(PO_2CH_2)\text{-}_{(L,D)}Xaa'\text{-}Yaa'\text{-}Zaa'\text{-}$$

ou répondant à l'une des formules (I) et (II) données ci-dessus.

Ces inhibiteurs ont la possibilité de bloquer in vivo la dégradation de nombreux peptides biologiques chez l'homme (somatostatine, bradykinine, angiotensine, neurotensine, substance P, dynorphine, VIP) et donc par là-même de potentialiser les effets biologiques de ces différents peptides. L'utilisation de ces produits in vivo débouche donc sur des applications pharmacologiques importantes impliquant ces peptides biologiques et leur dégradation par l'endopeptidase 24-15. D'autre part, cette même peptidase a été récemment impliquée dans la maladie d'Alzheimer et dans les étapes de maturation des protéines ras, protéines clés du développement de nombreuses formes de cancers. Il est à noter, que pour des produits similaires, des pseudopeptides phosphorés, des essais ont démontré que ces molécules in vivo, chez le chien, étaient effectivement capables d'inhiber la dégradation de la neurotensine, et ce pour des concentrations d'inhibiteur très faible comme il est décrit par Barelli, H.; Fox-Threlkeld, J.E.T..; Dive, V.; Daniel, E.E.; Vincent, J.P. & Checler, F.(1994)Br.J.Pharmacol. 112, 127.

Aussi, l'inhibition de l'endopeptidase 24-15 par les dérivés de l'invention, qui sont les plus puissants et les plus sélectifs reportés jusqu'à présent pour cette peptidase, a de nombreuses applications pharmacologiques, notamment comme analgésique et pour le traitement de l'hypothermie, de l'hypertension artérielle, du cancer et de la maladie d'Alzheimer.

D'autres caractéristiques et avantages de l'invention apparaîtront mieux à la lecture de la description qui suit donnée bien entendu à titre illustratif et non limitatif, en référence aux dessins annexés sur lesquels:

- les figures 1 à 3 déjà décrites illustrent la structure et l'interaction avec une enzyme d'un substrat d'enzyme ou d'un inhibiteur conforme à l'invention ; et
- la figure 4 illustre les différentes étapes de synthèse d'un dérivé de peptide conforme à l'invention.

Les exemples qui suivent illustrent la préparation de synthons et de dérivés de peptides conformes à l'invention et les propriétés des peptides obtenus.

## Exemple 1: Préparation du synthon Z-PheΨ[PO(OR$^3$)CH$_2$) GlyOH. (synthon n° 1)

### a) préparation de Z-PheΨ (PO(OR$^3$)CH$_2$)GlyO$_2$CH$_5$.

Pour cette préparation, on part du produit Z-PheΨ-(PO$_2$CH$_2$)GlyOC$_2$H$_5$ qui est obtenu conformément à l'exemple (composé n° 4) du document FR-A-2 676 059. Ce produit est dissous dans un mélange éthanol (20ml) et eau (5ml). Cette solution est ajoutée à une solution 1M de nitrate d'argent (4ml) sous agitation. Après 10 min, 20 ml d'eau sont ajoutés, puis l'alcool éthylique formé est évaporé sous vide. La phase aqueuse restante, contenant le précipité de sel d'argent, est refroidie dans un bain glace-eau pour 1 heure, puis le précipité est filtré, lavé avec de l'eau et séché en présence de P$_2$O$_5$ pour donner un solide (0,95g, rendement de 91% ). Ce sel d'argent (0,95g, 1,82mM) est suspendu dans une solution de chloroforme (15ml), à laquelle est ajouté le 1-adamantyle bromure (0,473g, 2,2 mM). Le mélange réactionnel est porté au reflux pendant 30min. Le précipité de bromure d'argent est éliminé par filtration, puis la solution filtrée est concentrée par évaporation. Le produit brut est purifié sur colonne de gel de silice en utilisant comme éluant un mélange chloroforme/ isopropanol (97:3) On obtient ainsi 0,66 g de produit pur Z-PheΨ(PO(O-R$^3$)CH2)GlyOC$_2$H$_5$ (rdt 81%) sous forme d'huile (Rf(1)=0,63; Rf(2)= 0,82 ; poids moléculaire=553,34).

### b) Préparation du synthon Z-Phe-Ψ (PO(OR$^3$)CH$_2$)GlyOH.

Le produit Z-PheΨ(PO(O-R$^3$)CH2)GlyOC$_2$H$_5$ obtenu en a) (0,55g, 1mM) est dissous dans l'éthanol (10mL), puis on ajoute goutte à goutte à cette solution lmL de soude 4M. Après 1 heure et demie, à température ambiante, l'éthanol est éliminé par évaporation sous vide. Le produit résiduel est dissous dans l'eau (30ml), cette solution est refroidi dans un bain glace-eau, puis acidifiée avec HCl 2M. Le produit solide qui précipite est extrait par l'acétate d'éthyle, lavé avec de l'eau, séché sur Na$_2$SO$_4$, puis concentré pour donner le produit solide Z-PheΨ(PO(O-R$^3$)CH2)GlyOH (0,46g, 89% rdt). Rf(2)=0,33 ; poids moléculaire= 525,34.

## Exemple 2 : Préparation du synthon Z-Phe-Ψ(PO(OR$^3$)CH$_2$) AlaOH (synthon n° 2)

Pour cette préparation, on suit le même mode opératoire que dans l'exemple 1 en partant du produit Z-PheΨ (PO$_2$CH$_2$)AlaOC$_2$H$_5$ obtenu de la même façon que le produit Z-PheΨ(PO$_2$CH$_2$)GlyOC$_2$H$_5$.
Les caractéristiques des produits obtenues sont les suivantes :

Z-Phe-Ψ(PO(OR$^3$)CH$_2$)AlaOC$_2$H$_5$: Rf(1)=0,66 ; poids moléculaire=566,35.
Z-Phe-Ψ(PO(OR$^3$)CH$_2$)AlaOH:Rf(1)=0,42; poids moléculaire=538,35.

## Exemple 3 : Préparation du synthon Z-Phe-Ψ (PO(OR$^3$)CH$_2$)LeuOH (synthon n°3)

On suit le même mode opératoire que dans l'exemple 1 pour préparer ce synthon à partir du produit Z-PheΨ (PO$_2$CH$_2$)LeuOC$_2$H$_5$ obtenu de la même façon que Z-Phe-Ψ-(PO$_2$CH$_2$)GlyOC$_2$H$_5$.
Les caractéristiques des produits obtenus sont les suivantes.

Z-PheΨ(PO(OR$^3$)CH$_2$)LeuOC$_2$H$_5$: Rf(1)=0,64 ; poids moléculaire = 610,1.
Z-PheΨ(PO(OR$^3$)CH$_2$)LeuOH:Rf(1)=0,52 ; poids moléculaire = 582,1.

## Exemple 4 : Préparation de Z-PheΨ(PO$_2$CH$_2$)GlyArgMetOH (peptide n° 1).

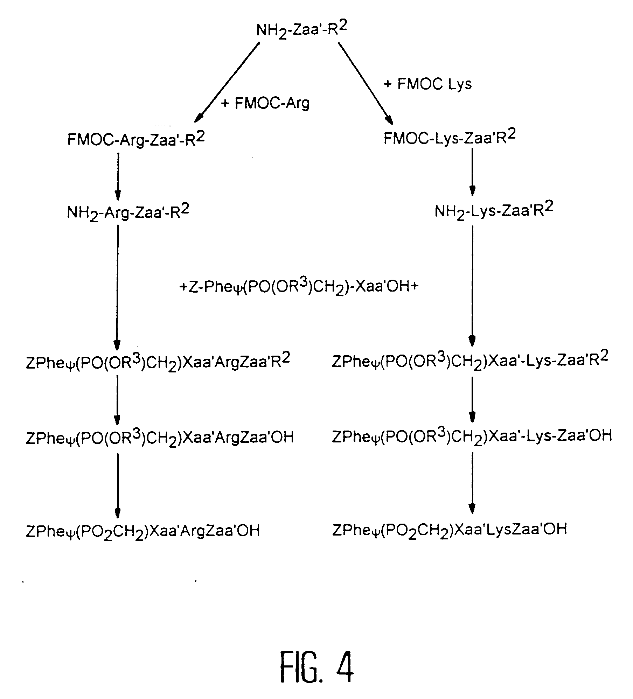

Pour préparer ce peptide, on utilise un mode de synthèse en phase solide décrit sur la figure 4 en fixant tout d'abord sur la phase solide l'acide aminé NH$_2$-MetOH. La phase solide est constituée par une résine 2-Clorotrityle et on utilisant 50 μmole de méthionine pour 50 à 100 mg de résine sèche.

### a) préparation de FMOC-Arg-Zaa'-R$^2$ avec Zaa' représentant Met, R$^2$ représentant la résine 2-Clorotrityle et FMOC représentant 9-Fluorenylméthyloxycarbonyle.

Tout d'abord on laisse gonfler la résine avec la méthionine fixée dessus, dans du diméthylformamide pendant 15 minutes, puis on réalise un couplage avec 2 à 4 équivalents de FMOC-Arg en utilisant pour ce couplage 2 à 4 équivalents de 2(1H Benzotriazol-l-yl)1,1,3,3 tétraméthyluronium hexafluorophosphate (HBTU), 2,5 à 5 équivalents de diisopropylamine (DIPEA) et 1 à 1,5 ml de diméthylformamide DMF (NMP) en réalisant le couplage en 30 à 60 minutes.

En fin d'opération, on rince avec 2 fois 2 ml de DMF, 1 fois 4 ml de dichlorométhane (DCM), 1 fois 2 ml d'isopropanol et 6 fois 2 ml de DMF.

b) préparation de $NH_2$-Arg-Zaa'-$R^2$.

Dans cette étape, on réalise un clivage du groupement FMOC avec 3 fois 2,5 ml respectivement pendant 5,5 et 10 minutes d'un mélange pipéridine/DMF (1:1). Après cette opération, on rince avec 2 fois 2 ml de DMF, 1 fois 4 ml de DCM, 1 fois 2 ml d'isopropanol et 6 fois 2 ml de DMF.

c) préparation de Phe-$\Psi$ $(PO(OR^3)CH_2)$Xaa'-Arg-Zaa'-$R^2$, avec Xaa'=Gly.

Pour cette étape, on utilise le synthon n° 1 obtenu dans l'Exemple 1 et on le couple avec le produit $NH_2$-Arg-Met-$R^2$ obtenu dans l'étape précédente.

Pour ce couplage, on utilise 1,5 équivalent du synthon n° 1, 3 équivalents de HBTU, 4 équivalents de DIPEA, et 1,5 ml de DMF en effectuant la réaction pendant 60 minutes. On rince ensuite avec 2 fois 2 ml de DMF, 1 fois 4 ml de DCM, 1 fois 2 ml d'isopropanol et 6 fois 2 ml de DMF.

d) préparation de Z-Phe$\Psi$ $(PO(OR^3)CH_2)$Xaa'-Arg-Zaa'OH.

Dans cette étape, on sépare le peptide de la phase solide $R^2$ en réalisant le clivage avec 5 ml d'un mélange d'acide acétique, de trifluoroéthanol (TFE) et de DCM (2:2:6). Après 120 minutes de contact, on rince avec 10 ml de DCM et on sèche à sec.

e) préparation du peptide n° 1.

Dans cette étape, on élimine le groupe adamantyle $R^3$ en opérant de la façon suivante.

On met en contact le peptide obtenu dans l'étape précédente avec 2,5 ml d'un mélange comprenant 50 à 60 % d'acide trifluoracétique TFA, 5 % de phénol, 5 % d'eau, 5 % de thioanisol, 2,5 % d'éthaneditiol et 42,5 à 32,5 % de DCM, en opérant pendant une durée de 40 à 180 minutes. On sèche ensuite à sec, puis on traite avec 3 équivalents de bicarbonate de sodium dans 2 ml d'eau et on extrait les impuretés dans l'éther diéthylique. On soumet ensuite le produit obtenu à une lyophilisation.

Dans l'étape de clivage, on peut utiliser, au lieu du mélange décrit précédemment 2,5 ml d'un mélange de 50 à 60 % de TFA, 5 % d'eau et 45 à 35 % de DCM.

Le peptide n° 1 obtenu présente les caractéristiques suivantes :

Rf = 0,7 (système propanol/eau 64:36)

Chromatographie liquide à haute performance (HPLC) : temps de rétention de 19,5 min ;

gradient linéaire de 28 min : éluant A (Solution aqueuse à 0,1 % d'acide trifluoracétique TFA) et éluant B (65 % d'acétonitrile dans solution aqueuse à 0,1 % de TFA)

**Exemple 5** :

Dans cet exemple, on suit le même mode opératoire que dans l'exemple 4 pour préparer les peptides n° 2 à 21 données dans le tableau 1 annexé.

**Exemple 6** :

Dans cet exemple, on teste l'activité biologique des peptides n° 1 à 21 pour déterminer leur effet inhibiteur vis-à-vis de l'endopeptidase 24-15 en opérant de la façon suivante :

On incube pendant 1 heure à 37°C, 2 nmole (20 µmole/l) de neurotensine qui est un substrat connu de l'endopeptidase 24.15, avec 7 µg d'endopeptidase 24.15 purifié dans un volume final de 100 µl du tampon Tris-HCl, 50 mM, pH 7,5, en l'absence d'inhibiteur (témoin) ou en présence du peptide testé à des concentrations allant de $10^{-11}$ à $10^{-7}$ mole/l. Après 1 heure d'incubation, on analyse les solutions par HPLC. A partir de cette analyse, on détermine la constante d'inhibition Ki du peptide testé.

Les résultats obtenus sont donnés dans le tableau 1.

**Exemple 7 :**

Dans cet exemple, on étudie l'effet inhibiteur des peptides de l'invention vis-à-vis de l'endopeptidase 24-16.

Dans ce but, on incube pendant 1 heure à 37°C, 2 nmole (20 µmole/l) de neurotensine, qui est un substrat connu de l'endopeptidase 24-16, avec 8 µg d'endopeptidase 24-16 purifié dans un volume final de 100 µl de tampon tris-HCl, 50mM, pH 7,5, en l'absence de peptide (témoin) ou en présence du peptide testé à des concentrations allant de $10^{-11}$ à $10^{-7}$ mole/l. Après 1 heure d'incubation, on analyse les solutions par HPLC et on détermine la constante d'inhibition Ki du peptide testé.

Les résultats obtenus sont donnés dans le tableau 1.

Dans ce tableau, on a donné également la sélectivité du peptide vis-à-vis de l'endopeptidase 24-16, soit le rapport $Ki_{24-16} / Ki_{24-15}$.

Les résultats du tableau 1 montrent que l'on obtient des peptides très efficaces, en particulier lorsque le dernier acide aminé du peptide est Met, Nle, Ala, Phe, Tyr, Leu ou Gln.

En effet, avec ces peptides n° 1 à 7, on obtient des inhibiteurs plus efficaces que l'inhibiteur connu Cpp-Ala Pro-Phe-pAb qui possède une constante d'inhibition de 7nM vis-à-vis de l'endopeptidase 24-15 de rat, comme il est décrit par Dando et al dans Biochem. J. 294, 1993, p. 451-457.

## Exemple 8 :

Dans cet exemple, on prépare des peptides de formule Z-PheΨ(PO$_2$CH$_2$)Ala-Lys-Zaa'OH en suivant le même mode opératoire que dans l'exemple 4, mais en utilisant le synthon n° 2.

On teste ensuite l'effet inhibiteur de ces peptides vis-à-vis de l'endopeptidase 24-15 et éventuellement de l'endopeptidase 24-16 en suivant le même mode opératoire que dans les exemples 6 et 7.

Les résultats obtenus sont donnés dans le tableau 2. Ces résultats montrent que les peptides n° 22 à 34 sont des inhibiteurs très puissants de l'endopeptidase 24-15.

A titre comparatif, on peut noter que des mélanges de peptides du type Z-PheΨ (PO$_2$CH$_2$)Ala-Pro-Zaa'OH et Z-PheΨ(PO$_2$CH$_2$)Ala-Nle-Zaa'OH, par rapport à des mélanges du type :

Z-PheΨ[PO$_2$ CH$_2$]Ala Arg Zaa' ou Z-PheΨ[PO$_2$ CH$_2$]Ala-Lys Zaa'dans lesquels Zaa' représente les 20 acides aminés naturels, ont des constantes d'inhibition Ki bien plus faibles et présentent en outre une très faible sélectivité vis-à-vis de l'inhibition de l'endopeptidase 24-16.

| PEPTIDE | Ki24-15 (nM) | Ki24-16 (nM) | SELECTIVITE |
|---|---|---|---|
| Z-PheΨ(PO$_2$CH$_2$)Ala-Pro-Zaa'OH | 35 | 60 | 1,7 |
| Z-PheΨ(PO$_2$CH$_2$)Ala-Nle-Zaa'OH | 52 | 330 | 6,3 |
| Z-PheΨ(PO$_2$CH$_2$)Ala-Lys-Zaa'OH | 3,5 | 900 | 257 |
| Z-PheΨ(PO$_2$CH$_2$)Ala-Arg-Zaa'OH | 3,5 | 1600 | 457 |

Ainsi, on remarque que le remplacement de Arg ou Lys par Pro ou Nle donne des inhibiteurs qui ne sont plus très sélectifs vis-àvis de l'endopeptidase 24-15.

## Exemple 9 :

Dans cet exemple, on prépare les peptides n° 42, 43 et 44, en suivant le même mode opératoire que dans l'exemple 4, mais en partant du synthon n° 2 pour les peptides n° 42 et 43, et du synthon n° 3 pour le peptide n° 44.

On teste les propriétés inhibitrices des peptides obtenus vis-à-vis de l'endopeptidase 24-15 et de l'endopeptidase 24-16 en suivant le même mode opératoire que dans les exemples 6 et 7.

Les résultats obtenus sont donnés dans le tableau 3.

Dans ce tableau, on a regroupé également les résultats obtenus avec les peptides n° 1, n° 4 et n°25 qui comportent le même acide aminé en bout de chaîne que ceux des peptides n° 42, 43 et 44.

Les résultats de ce tableau montrent que le peptide n° 43 Z-$_{(D-L)}$PheΨ(PO$_2$CH$_2$)(D,L)Ala-Arg-PheOH est l'inhibiteur le plus puissant qui soit connu pour l'endopeptidase 24-15, tout en étant extrêmement sélectif. (Facteur de sélectivité vis-à-vis de l'endopeptidase 24-16 de 3335). Par exemple, l'inhibiteur connu Cpp-Ala-Pro-Phe-pAb possède un Ki de 7nM vis-à-vis de l'endopeptidase 24-15 de rat, alors que dans les mêmes conditions le mélange d'inhibiteurs Z-$_{(D-L)}$PheΨ(PO$_2$CH$_2$)$_{(D,L)}$Ala-Arg-PheOH, comprenant 4 diastéréoisomères, possède un Ki de 0,16 nM. S'agissant d'un mélange, il peut être attendu que l'un de ces diastéréoisomères ait un Ki de l'ordre de 40 pM, soit une différence d'affinité avec le produit Cpp-Ala-Pro-pAb de l'ordre de 175. De même, le peptide n° 42 Z-$_{(D-L)}$PheΨ(PO$_2$CH$_2$)$_{(D,L)}$Ala-Arg-MetOH constitue le mélange le plus puissant des peptides testés selon l'invention avec un Ki de 0,067nM. L'un des diastéréoisomères de ce mélange possède sans doute une affinité de l'ordre de 15 pM, soit une puissance augmentée par un facteur 440.

Par ailleurs, tous ces peptides sont très sélectifs pour l'endopeptidase 24-15.

Enfin, il est important de noter que la spécifité entre les endopeptidases 24-15 de différentes espèces (rat, poulet, homme) ne variant pas, les peptides de l'invention sont donc des inhibiteurs puissants de l'endopeptidase 24-15 de l'homme.

**TABLEAU 1**

| PEPTIDE | FORMULE | Ki24-15 (nM) | Ki24-16 (nM) | SELECTIVITE |
|---|---|---|---|---|
| n° 1 | Z-PheΨ($PO_2CH_2$)Gly-Arg-Met | 0,35 | 132 | 377 |
| n° 2 | Z-PheΨ($PO_2CH_2$)Gly-Arg-Nle | 1,6 | 213 | 135 |
| n° 3 | Z-PheΨ($PO_2CH_2$)Gly-Arg-Ala | 2,2 | 201 | 91 |
| n° 4 | Z-PheΨ($PO_2CH_2$)Gly-Arg-Phe | 2,7 | 1103 | 409 |
| n° 5 | Z-PheΨ($PO_2CH_2$)Gly-Arg-Tyr | 3,6 | 596 | 165 |
| n° 6 | Z-PheΨ($PO_2CH_2$)Gly-Arg-Leu | 5,0 | 846 | 169 |
| n° 7 | Z-PheΨ($PO_2CH_2$)Gly-Arg-Gln | 6,1 | 690 | 113 |
| n° 8 | Z-PheΨ($PO_2CH_2$)Gly-Arg-Val | 9,3 | 1128 | 121 |
| n° 9 | Z-PheΨ($PO_2CH_2$)Gly-Arg-Ile | 9,6 | 589 | 61 |
| n°10 | Z-PheΨ($PO_2CH_2$)Gly-Arg-Trp | 13,1 | 2664 | 203 |

TABLEAU 1 (suite)

| n°11 | Z-PheΨ(PO$_2$CH$_2$)Gly-Arg-Gly | 13,8 | 1661 | 120 |
|---|---|---|---|---|
| n°12 | Z-PheΨ(PO$_2$CH$_2$)Gly-Arg-Arg | 14,0 | 2037 | 145 |
| n°13 | Z-PheΨ(PO$_2$CH$_2$)Gly-Arg-Asn | 19,5 | 1380 | 71 |
| n°14 | Z-PheΨ(PO$_2$CH$_2$)Gly-Arg-His | 23,2 | 3009 | 129 |
| n°15 | Z-PheΨ(PO$_2$CH$_2$)Gly-Arg-Ser1 | 28,8 | 2057 | 87 |
| n°16 | Z-PheΨ(PO$_2$CH$_2$)Gly-Arg-Ser2 | 32,0 | 1379 | 43 |
| n°17 | Z-PheΨ(PO$_2$CH$_2$)Gly-Arg-Thr | 35,2 | 3636 | 103 |
| n°18 | Z-PheΨPO$_2$CH$_2$)Gly-Arg-Lys | 138 | 25700 | 186 |
| n°19 | Z-PheΨ(PO$_2$CH$_2$)Gly-Arg-Glu | 163 | 8149 | 50 |
| n°20 | Z-PheΨ(PO$_2$CH$_2$)Gly-Arg-Asp | 352 | 20373 | 58 |
| n°21 | Z-PheΨ(PO$_2$CH$_2$)Gly-Arg-Pro | 3520 | 36670 | 10 |

TABLEAU 2

| PEPTIDE | FORMULE | Ki24-15 (nM) | Ki24-16 (nM) | SELECTIVITE |
|---|---|---|---|---|
| n°22 | Z-PheΨ(PO$_2$CH$_2$)Ala-Lys-Met | 0,115 | 225 | 1957 |
| n°23 | Z-PheΨ(PO$_2$CH$_2$)Ala-Lys-Nle | 0,63 | 162 | 257 |
| n°24 | Z-PheΨ(PO$_2$CH$_2$)Ala-Lys-Ala | 0,83 | 436 | 525 |
| n°25 | Z-PheΨ(PO$_2$CH$_2$)Ala-Lys-Phe | 1,00 | 1652 | 1652 |
| n°26 | Z-PheΨ(PO$_2$CH$_2$)Ala-Lys-Leu | 1,82 | 893 | 491 |
| n°27 | Z-PheΨ(PO$_2$CH$_2$)Ala-Lys-Ile | 1,94 | 1265 | 652 |
| n°28 | Z-PheΨ(PO$_2$CH$_2$)Ala-Lys-Gln | 2,18 | | |
| n°29 | Z-PheΨ(PO$_2$CH$_2$)Ala-Lys-Tyr | 2,30 | | |
| n°30 | Z-PheΨ(PO$_2$CH$_2$)Ala-Lys-Val | 2,60 | | |
| n°31 | Z-PheΨ(PO$_2$CH$_2$)Ala-Lys-Arg | 3,17 | | |

TABLEAU 2 (suite)

| | | | | |
|---|---|---|---|---|
| n°32 | Z-PheΨ(PO$_2$CH$_2$)Ala-Lys-Asn | 4,90 | | |
| n°33 | Z-PheΨ(PO$_2$CH$_2$)Ala-Lys-Gly | 5,10 | | |
| n°34 | Z-PheΨ(PO$_2$CH$_2$)Ala-Lys-Trp | 5,78 | | |
| n°35 | Z-PheΨ(PO$_2$CH$_2$)Ala-Lys-His | 7,13 | | |
| n°36 | Z-PheΨ(PO$_2$CH$_2$)Ala-Lys-Thr | 10,1 | | |
| n°37 | Z-PheΨ(PO$_2$CH$_2$)Ala-Lys-Ser | 11,9 | | |
| n°38 | Z-PheΨ(PO$_2$CH$_2$)Ala-Lys-Lys | 26,9 | | |
| n°39 | Z-PheΨ(PO$_2$CH$_2$)Ala-Lys-Glu | 53,5 | | |
| n°40 | Z-PheΨ(PO$_2$CH$_2$)Ala-Lys-Asp | 109,0 | | |
| n°41 | Z-PheΨ(PO$_2$CH$_2$)Ala-Lys-Pro | 1030 | | |

TABLEAU 3

| PEPTIDE | FORMULE | Ki24-15 (nM) | Ki24-16 (nM) | SELECTIVITE |
|---|---|---|---|---|
| n° 42 | $Z-Phe\Psi(PO_2CH_2)Ala-Arg-Met$ | 0,067 | 88 | 1312 |
| n° 1 | $Z-Phe\Psi(PO_2CH_2)Gly-Arg-Met$ | 0,35 | 132 | 377 |
| n° 43 | $Z-Phe\Psi(PO_2CH_2)Ala-Arg-Phe$ | 0,158 | 527 | 3335 |
| n° 4 | $Z-Phe\Psi(PO_2CH_2)Gly-Arg-Phe$ | 2,7 | 1103 | 409 |
| n° 44 | $Z-Phe\Psi(PO_2CH_2)Leu-Arg-Phe$ | 14 | 2285 | 164 |
| n° 25 | $Z-Phe\Psi(PO_2CH_2)Ala-Lys-Phe$ | 1 | 1652 | 1652 |

**Revendications**

1. Dérivé de peptide comportant la séquence d'acides aminés suivante :

$$- Phe\ \Psi(PO_2CH_2)-_{(L,D)}Xaa'-Yaa'-Zaa'-$$

dans laquelle

$\Psi(PO_2CH_2)$ indique que la liaison peptidique (CONH) a été remplacée par la liaison phosphinique $(PO_2CH_2)$,

Xaa' et Zaa' qui peuvent être identiques ou différents, représentent chacun un acide aminé naturel ou un pseudo-acide aminé, et
Yaa' représente Arg ou Lys.

2. Dérivé de peptide répondant à l'une des formules :

$$Z\text{-}_{(L,D)}Phe\ \Psi(PO_2CH_2)\text{-}_{(L,D)}Xaa'\text{-}Arg\text{-}Zaa'OR^1 \qquad (I)$$

et

$$Z\text{-}_{(L,D)}Phe\ \Psi(PO_2CH_2)\text{-}_{(L,D)}Xaa'\text{-}Lys\text{-}Zaa'OR^1 \qquad (II)$$

dans lesquelles

Z représente le groupe acétyle ou benzyloxycarbonyle,
$\Psi(PO_2CH_2)$ indique que la liaison peptidique (CO-NH) a été remplacée par la liaison phosphinique $(PO_2CH_2)$,
Xaa' et Zaa' qui peuvent être identiques ou différents, représentent chacun un acide aminé naturel ou un pseudo acide aminé, et
$R^1$ représente un atome d'hydrogène, $NH_4^+$ ou un métal pharmaceutiquement acceptable.

3. Dérivé de peptide selon l'une quelconque des revendications 1 et 2, caractérisé en ce que Xaa' représente Gly, Ala ou Leu.

4. Dérivé de peptide selon l'une quelconque des revendications 1 à 3, caractérisé en ce que Zaa' représente Met, Nle, Ala ou Phe.

5. Dérivé de peptide répondant à la formule :

$$Z\text{-}_{(L,D)}Phe\ \Psi(PO_2CH_2)\text{-}_{(L,D)}Xaa'\ Lys\ Zaa'OR^1 \qquad (II)$$

dans laquelle

Z représente le groupe benzyloxycarbonyle,
$\Psi(PO_2CH_2)$ indique que la liaison peptidique (CO-NH) a été remplacée par la liaison phosphinique $(PO_2\text{-}CH_2)$,
Xaa' représente Gly, Ala ou Leu,
Zaa' représente Met, NLe, Ala, Phe, Leu ou Ile, et
$R^1$ représente un atome d'hydrogène, $NH_4^+$ ou un métal pharmaceutiquement acceptable.

6. Dérivé de peptide selon la revendication 5, caractérisé en ce que
Xaa' représente Ala, Zaa' représente Met et $R^1$ représente un atome d'hydrogène.

7. Dérivé de peptide de formule

$$Z\text{-}_{(L,D)}Phe\ \Psi(PO_2CH_2)\text{-}_{(L,D)}Gly\text{-}Arg\text{-}Met\text{-}OH$$

dans laquelle Z représente le groupe benzyloxycarbonyle, et
$\Psi(PO_2CH_2)$ indique que la liaison peptidique (CO-NH) a été remplacée par la liaison phosphinique $(PO_2\text{-}CH_2)$.

8. Dérivé de peptide de formule

$$Z\text{-}_{(L,D)}Phe\ \Psi(PO_2CH_2)\text{-}_{(L,D)}Ala\text{-}Arg\text{-}Zaa'OH$$

dans laquelle
Z représente le groupe benzyloxycarbonyle,

- $\Psi(PO_2CH_2)$ indique que la liaison peptidique (CO-NH) a été remplacée par la liaison phosphinique $(PO_2CH_2)$ et
- Zaa' représente Met ou Phe.

9. Procédé de préparation d'un dérivé de peptide répondant à la formule

$$Z\text{-}_{(L,D)}Phe\ \Psi(PO_2CH_2)\text{-}_{(L,D)}Xaa'\text{-}Arg\text{-}Zaa'OR \qquad (I)$$

dans laquelle

Z représente le groupe benzyloxycarbonyle,

$\Psi(PO_2CH_2)$ indique que la liaison peptidique (CO-NH) a été remplacée par la liaison phosphinique $(PO_2CH_2)$,

Xaa' et Zaa' qui peuvent être identiques ou différents, représentent chacun un acide aminé naturel ou synthétique, et

$R^1$ représente un atome d'hydrogène, $NH_4^+$ ou un métal pharmaceutiquement acceptable,

caractérisé en ce qu'il consiste à coupler le dipeptide fixé sur une phase solide, de formule

$$NH_2\text{-Arg-Zaa'R}^2$$

dans laquelle Zaa' a la signification donnée ci-dessus et $R^2$ est la phase solide, avec le dipeptide de formule

$$Z\text{-}_{(L,D)}Phe\ \Psi(PO(OR^3)CH_2)\text{-}_{(L,D)}Xaa'OH$$

dans laquelle Z, $\Psi(PO_2CH_2)$ et Xaa' ont les significations données ci-dessus, et $R^3$ représente le groupe adamantyle, puis à séparer le dérivé de peptide de formule

$$Z\text{-}_{(L,D)}Phe\ \Psi(PO(OR^3)CH_2)\text{-}_{(L,D)}Xaa'\text{-Arg-Zaa'R}^2$$

de la phase solide $R^2$, et à éliminer le groupe $R^3$ par un traitement acide.

**10.** Procédé de préparation d'un dérivé de peptide de formule (II)

$$Z\text{-}_{(L,D)}Phe\ \Psi(PO_2CH_2)\text{-}_{(L,D)}Xaa'\text{-Lys-Zaa'OR}^1 \tag{II}$$

   dans laquelle

Z représente le groupe benzyloxycarbonyle,

$\Psi(PO_2CH_2)$ indique que la liaison peptidique (CO-NH) a été remplacée par la liaison phosphinique $(PO_2CH_2)$,

Xaa' et Zaa' qui peuvent être identiques ou différents, représentent chacun un acide aminé naturel ou synthétique, et

$R^1$ représente un atome d'hydrogène, $NH_4$ ou un métal pharmaceutiquement acceptable

caractérisé en ce qu'il consiste à coupler le dipeptide fixé sur une phase solide, de formule

$$NH_2\text{-Lys-Zaa'R}^2$$

dans laquelle Zaa' a la signification donnée ci-dessus et $R^2$ est la phase solide, avec le dipeptide de formule
$Z\text{-}_{(L,D)}Phe\ \Psi(PO(OR^3)CH_2)\text{-}_{(L,D)}Xaa'OH$
dans laquelle Z, $\Psi(PO_2CH_2)$ et Xaa' ont les significations données ci-dessus, et $R^3$ représente le groupe adamantyle, puis à séparer le dérivé de dipeptide de formule

$$Z\text{-}_{(L,D)}Phe\ \Psi(PO(OR^3)CH_2)\text{-}_{(L,D)}Xaa'\text{-Lys-Zaa'R}^2$$

de la phase solide $R^2$, et à éliminer le groupe $R^3$ par un traitement acide.

**11.** Composition pharmaceutique comprenant un inhibiteur de l'endopeptidase 24-15, caractérisé en ce que cet inhibiteur est un dérivé de peptide selon l'une quelconque des revendications 1 à 8.

**12.** Composition pharmaceutique selon la revendication 11 utilisable comme agent hypothermiant, agent analgésique, ou agent de traitement de l'hypertension artérielle, du cancer ou de la maladie d'Alzheimer.

FIG.1

FIG. 2

FIG. 3

NH$_2$-Zaa'-R$^2$

+ FMOC-Arg

+ FMOC Lys

FMOC-Arg-Zaa'-R$^2$

FMOC-Lys-Zaa'R$^2$

NH$_2$-Arg-Zaa'-R$^2$

NH$_2$-Lys-Zaa'R$^2$

+Z-Pheψ(PO(OR$^3$)CH$_2$)-Xaa'OH+

ZPheψ(PO(OR$^3$)CH$_2$)Xaa'ArgZaa'R$^2$

ZPheψ(PO(OR$^3$)CH$_2$)Xaa'-Lys-Zaa'R$^2$

ZPheψ(PO(OR$^3$)CH$_2$)Xaa'ArgZaa'OH

ZPheψ(PO(OR$^3$)CH$_2$)Xaa'-Lys-Zaa'OH

ZPheψ(PO$_2$CH$_2$)Xaa'ArgZaa'OH

ZPheψ(PO$_2$CH$_2$)Xaa'LysZaa'OH

FIG. 4

EP 0 725 075 A1

## DOCUMENTS CONSIDERES COMME PERTINENTS

Office européen
des brevets

RAPPORT DE RECHERCHE EUROPEENNE

Numero de la demande
EP 96 40 0229

| Catégorie | Citation du document avec indication, en cas de besoin, des parties pertinentes | Revendication concernée | CLASSEMENT DE LA DEMANDE (Int.Cl.6) |
|---|---|---|---|
| P,X | BR. J. PHARMACOL. (1995), 115(6), 1053-63 CODEN: BJPCBM;ISSN: 0007-1188, XP000571651 VINCENT, BRUNO ET AL: "Phosphorus-containing peptides as mixed inhibitors of endopeptidase 3.4.24.15 and 3.4.25.16: effect on neurotensin degradation in vitro and in vivo" * page 1059, colonne de droite, alinéa 3 - page 1062, colonne de droite, alinéa 1; tableau 1 * | 1-11 | C07K5/03 A61K38/07 |
| X | EP-A-0 276 436 (HOFFMANN LA ROCHE) 3 Août 1988 * revendications; exemples * | 1 | |
| X | WO-A-93 14112 (MERCK & CO INC) 22 Juillet 1993 * revendications; exemples * | 1 | |
| D,A | EP-A-0 565 450 (COMMISSARIAT ENERGIE ATOMIQUE) 13 Octobre 1993 * revendications; exemples 10,11,17 * | 1-3,11, 12 | |
| D,A | DE-A-40 36 130 (COMMISSARIAT ENERGIE ATOMIQUE) 16 Mai 1991 * revendications; exemple 5 * | 1-3,10, 11 | DOMAINES TECHNIQUES RECHERCHES (Int.Cl.6) C07K A61K |
| D,A | BIOCHEM. J. (1992), 287(2), 621-5 CODEN: BIJOAK;ISSN: 0306-3275, XP000571638 BARELLI, HELENE ET AL: "Potent inhibition of endopeptidase 24.16 and endopeptidase 24.15 by the phosphonamide peptide N-(phenylethylphosphonyl)-Gly-L-Pro-L-aminohexanoic acid" * page 622, colonne de gauche, alinéa 2 - alinéa 5 * * page 624, colonne de gauche, alinéa 2 - colonne de droite, dernier alinéa * | 1-3,10, 11 | |

-/--

Le présent rapport a été établi pour toutes les revendications

| Lieu de la recherche | Date d'achèvement de la recherche | Examinateur |
|---|---|---|
| LA HAYE | 30 Mai 1996 | Fuhr, C |

Office européen
des brevets

## RAPPORT DE RECHERCHE EUROPEENNE

Numero de la demande

EP 96 40 0229

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | BIOCHEM. J. (1995), 308(1), 145-50 CODEN: BIJOAK;ISSN: 0264-6021, XP000571639 KNIGHT, C. GRAHAM ET AL: "Thimet oligopeptidase specificity: evidence of preferential cleavage near the C-terminus and product inhibition from kinetic analysis of peptide hydrolysis" * page 149, colonne de gauche, alinéa 2 - colonne de droite, dernier alinéa; tableau 5 * | 1-11 | |
| A | JOURNAL OF MEDICINAL CHEMISTRY, vol. 36, no. 10, 14 Mai 1993, WASHINGTON US, pages 1369-1379, XP002004272 S. DOULOT ET AL.: "Synthesis and analgesic effect of N-[3-[(Hydroxyamino)carbonyl]-1-oxo-2(R)-b enzylporpyl]-L-isoleucyl-L-leucine, a New Potent Inhibitor of Multiple Neurotensin/Neuromedin N Degrading Enzymes" * page 1369, colonne de droite, alinéa 2 - page 1375, colonne de gauche, alinéa 2; tableau II * | 1-11 | DOMAINES TECHNIQUES RECHERCHES (Int.Cl.6) |

Le présent rapport a été établi pour toutes les revendications

| Lieu de la recherche | Date d'achèvement de la recherche | Examinateur |
|---|---|---|
| LA HAYE | 30 Mai 1996 | Fuhr, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)